# EUROPÄISCHE PATENTSCHRIFT

(11) **EP 2 666 452 B1**
(45) Veröffentlichungstag und Bekanntmachung des Hinweises auf die Patenterteilung: **30.11.2016**
(21) Anmeldenummer: 13167806.2
(22) Anmeldetag: 15.05.2013
(51) Int. Cl.: A61G 12/00, A47B 96/04, A61B 50/13, F16M 11/00

(54) **Stationswagen, insbesondere Visiten- und Pflegewagen**
Trolley, in particular ward trolley and care trolley
Voiture break, notamment voiture de visites et de soins

(30) Priorität: 22.05.2012 DE 202012101860 U
(43) Veröffentlichungstag der Anmeldung: 27.11.2013
(73) Patentinhaber: Optiplan Gesellschaft für optische Planungsgeräte mit beschränkter Haftung, 40489 Düsseldorf (DE)
(72) Erfinder: Wagner, Kai, 40489 Düsseldorf (DE); Lerner, Sabine, 40489 Düsseldorf (DE)
(74) Vertreter: Stenger Watzke Ring

(56) Entgegenhaltungen:
- DE-U1- 20 005 770
- US-A1- 2005 001 395

## Beschreibung

Die Erfindung betrifft einen Stationswagen, insbesondere einen Visiten- und Pflegewagen für Krankenhäuser, Pflegeheime und sonstige Einrichtungen des Gesundheitswesens, mit einem fahrbar ausgebildeten Schrankteil, wobei das Schrankteil einen frontseitig zugänglichen Stauraum zur Aufnahme von Utensilien und einen in Beschickungsrichtung des Stauraums dahinterliegenden und vom Stauraum durch eine Zwischenwand getrennten Kabelraum aufweist, wobei der Stauraum ein Staufach bereitstellt, das einen Zugang zum Kabelraum aufweist.

Ein Stationswagen der eingangs genannten, d.h. gattungsgemäßen Art ist aus dem Stand der Technik an sich bekannt. Einen solchen Stationswagen offenbart beispielsweise die US 2005/0001395 A1. Dabei besteht die Besonderheit des vorbekannten Stationswagens darin, dass er über verstellbare und in ihrer Position veränderliche Trennwände verfügt, die es gestatten, eine Bestückung des Stationswagens mit Utensilien patientenbezogen zu individualisieren.

Weitere Stationswagen sind beispielsweise aus der DE 297 14 981, der DE 20 2005 013 115, der DE 20 2005 013 114, der DE 20 2008 015 373 sowie der DE 20 2011 051 973 bekannt geworden.

Die aus dem Stand der Technik bekannten Stationswagen haben sich im alltäglichen Praxiseinsatz bewährt. Sie sind aus dem Schrankteil gebildet, das einen Stauraum bereitstellt. Dabei kann der Stauraum unterteilt ausgebildet sein. Der Stauraum dient dazu, Utensilien aufnehmen zu können, wobei für einen besseren Zugriff auf die Utensilien Schubladen, Körbe und/oder dgl. Fächer vorgesehen sein können.

Unterseitig schließt das Schrankteil mit einer Bodenplatte ab. Diese ist unterseitig mit Tragrollen bestückt, womit der Stationswagen als solcher verfahrbar ist.

Oberseitig des Schrankteils ist zumeist eine Abdeckung vorgesehen, die im alltäglichen Praxiseinsatz beispielsweise als Schreibunterlage und/oder der Aufnahme von Patientenunterlagen dient.

Aus dem Stand der Technik sind auch solche Stationswagen bekannt geworden, die mit einer EDV-Anlage ausgerüstet sind und zu diesem Zweck über einen am Stationswagen angeordneten Monitor verfügen. Ein solcher Stationswagen ist beispielsweise aus der DE 20 2011 051 973 bekannt geworden.

Obgleich sich die aus dem Stand der Technik vorbekannten Stationswagen im alltäglichen Praxiseinsatz bewährt haben, besteht Verbesserungsbedarf, insbesondere mit Blick auf eine vereinfachte Handhabung. Es ist deshalb die **Aufgabe** der Erfindung, einen Stationswagen bereitzustellen, der in seiner Handhabung verbessert ist, und dies bei gleichzeitiger Gewährleistung erhöhter Hygiene- und Reinigungsanforderungen
Zur **Lösung** dieser Aufgabe wird mit der Erfindung ein Stationswagen der eingangs genannten Art vorgeschlagen, der sich auszeichnet durch ein am Schrankteil angeordnetes und von außen zugängliches Energiemanagementsystem, wobei das Energiemanagementsystem gekapselt ausgebildet und unterseitig einer Bodenplatte des Schrankteils angeordnet ist, und durch einen verschwenkbar am Schrankteil angeordneten Monitor, wobei der Monitor von einem Rohr getragen ist, das mit seinem Monitor entfernten Ende in den Kabelraum hineinragt, wobei der Monitor unter Zwischenordnung eines Gelenks am Rohr angeordnet ist, wobei das Gelenk durch eine Tülle abgedeckt ist.

Das Schrankteil nach dem erfindungsgemäßen Stationswagen verfügt in an sich bekannter Weise über einen Stauraum. Dieser dient der Aufnahme von Utensilien. In Ergänzung zum Stauraum ist ein Kabelraum vorgesehen, der vom Schrankteil bereitgestellt ist. Der vom Schrankteil insgesamt bereitgestellte Volumenraum ist demnach aus einem Stauraum einerseits und einem Kabelraum andererseits gebildet.

Dabei ist vorgesehen, dass der Kabelraum in Beschickungsrichtung des Schrankteils hinter dem Stauraum liegend ausgebildet ist. Der Stauraum und der Kabelraum sind durch eine Zwischenwand voneinander getrennt, so dass ein frontseitiger Zugriff auf den Kabelraum verwenderseitig nicht möglich ist. Insofern kann die Zwischenwand auch als Trennwand bezeichnet werden, die dazu dient, den Kabelraum vor einem frontseitigen Zugriff durch einen Verwender abzuschirmen.

Der Stauraum stellt ein Staufach bereit. Dieses Staufach verfügt über einen Zugang zum Kabelraum. Es besteht mithin eine Zugriffsmöglichkeit bzw. ein direkter Zugang zum Kabelraum über das vom Stauraum des Schrankteils bereitgestellte Staufach.

Die vorbeschriebene Ausgestaltung ermöglicht es, eine EDV-Anlage in optimierter Weise aufnehmen zu können, wobei auch unter Umständen störende Verkabelungen dauerhaft sicher untergebracht sind, was insbesondere die Handhabung vereinfacht. Zudem trägt die Entwicklung der gekapselten Ausführung des Monitors und der verdeckten Kabelführung den im Krankenhaus geforderten erhöhten Hygiene- und Reinigungsanforderungen Rechnung. Weiterhin sind etwaige Ausfälle, Beschädigungen und/oder dgl. durch Kabelbruch und/oder sich lösende Kabel weitestgehend ausgeschlossen, da der Kabelraum eine ordnungsgemäße und vor äußeren Einflüssen geschützte Anordnung und Aufbewahrung der Kabel der EDV-Anlage konstruktiv sicherstellt.

Das vom Stauraum bereitgestellte Staufach dient der Aufnahme des Rechners der EDV-Anlage. Es ist bevorzugter Weise in diesem Zusammenhang vorgesehen, dass das Staufach frontseitig, d.h. zugriffsseitig mittels einer Staufachtür ausgerüstet ist. Diese kann mittels eines Schlosses abschließbar ausgerüstet sein, so dass ein ungewollter Zugriff auf den vom Staufach aufgenommenen Rechner unterbunden ist.

Das Schrankteil trägt verschwenkbar einen Monitor. Dieser ist in an sich bekannter Weise über eine Kabelverbindung an den im Staufach angeordneten Rechner angeschlossen. Zum Zwecke der Kabelführung dient der in Beschickungsrichtung des Schrankteils hinter dem Stauraum ausgebildete Kabelraum. Zur Einführung der Kabel in den Kabelraum ist erfindungsgemäß ein Zugang vom Staufach zum Kabelraum vorgesehen. Bei diesem Zugang kann es sich um eine Öffnung oder einen Durchbruch in der den Stauraum vom Kabelraum trennenden Zwischenwand handeln.

Der Monitor wird vorzugsweise von einem aus Aluminium oder einer Aluminiumlegierung gebildeten Rohr getragen. Dabei ragt das monitorentferne Ende des Rohrs in den Kabelraum hinein. Dies gestattet es, die vom Rechner in den Kabelraum geführten Kabel von außen unzugänglich durch das den Monitor tragende Rohr bis zu den Anschlussbuchsen des Monitors zu führen. Auch das für den Monitor vorzusehende Stromversorgungskabel kann über das den Monitor tragende Rohr bis in den Kabelraum geführt werden.

Der Übergangsbereich zwischen Monitor einerseits und Rohr andererseits ist durch eine vorzugsweise aus Kunststoff gebildete Tülle oder Manschette abgedeckt sein. Dabei ist zur Anordnung des Monitors am Rohr ein ebenfalls von der Tülle oder der Manschette abgedecktes Gelenk vorgesehen sein, so dass der Monitor in seiner Neigung zum Rohr wahlweise ausgerichtet werden kann.

Gemäß einem besonderen Vorteil der Erfindung ist der Monitor höhenverstellbar am Schrankteil des Stationswagens angeordnet. Diese Höhenverstellung kann beispielsweise dadurch realisiert sein, dass das den Monitor tragende Rohr höhenverstellbar von einer Führung des Schrankteils gehalten ist. Eine Höhenverstellung des Monitors kann also durch eine entsprechende Höhenverstellung des Rohres bewirkt werden. Dabei ist in vorteilhafter Weise vorgesehen, die höhenverstellbare Aufnahme des Rohres innerhalb des Kabelraums bereitzustellen, womit nicht nur die Kabel der EDV-Anlage, sondern auch die Monitorhöhenverstellung vom Kabelraum sicher und vor äußeren Einflüssen geschützt aufgenommen ist.

Der mit der Erfindung vorgeschlagene Stationswagen kann insgesamt als gekapselt ausgebildet bezeichnet werden. Insbesondere die Monitorhöhenverstellung sowie die Kabelführung sind in einem separat ausgebildeten und vom Stauraum des Schrankteils getrennt ausgebildeten Kabelraum untergebracht. Ein Zugriff von außen ist nicht ohne Weiteres möglich, und auch eine Beeinträchtigung im alltäglichen Praxiseinsatz durch freiliegende Kabel ist vermieden.

Ein Zugriff auf den Kabelraum ist gemäß einem weiteren Merkmal der Erfindung von der Rückseite des Schrankteils her möglich. Zu diesem Zweck verfügt das Schrankteil über eine entnehmbare Rückwand. Diese Rückwand kann verwenderseitig sowohl im Falle beispielsweise einer Ersteinrichtung und/oder im Falle einer Reparatur und/oder Nachrüstung entfernt werden, womit der Zugriff auf den Kabelraum und damit auf die davon aufgenommene Verkabelung möglich ist. Dabei kann die Rückwand abschließbar ausgebildet sein, so dass ein manipulativer Zugriff durch Nichtberechtigte unterbunden ist.

Gemäß einem weiteren Merkmal der Erfindung ist vorgesehen, dass in die Rückwand ein 230V Kaltgerätstecker integriert ist. Dieser dient dem Anschluss eines Stromversorgungskabels und/oder eines Ladegerätes. Der Kaltgerätestecker ist seinerseits an die EDV-Anlage und/oder ein Energiemanagementsystem angeschlossen, so dass über diesen zentralen Anschluss eine Stromversorgung ermöglicht ist.

Die Einschalt- sowie etwaige Steuerfunktionen erfolgen verwenderseitig über den am Schrankteil angeordneten Monitor, so dass im bestimmungsgemäßen Verwendungsfall ein verwenderseitiger Zugriff auf den vom Staufach aufgenommenen Rechner nicht erforderlich ist. Es kann darüber hinaus vorgesehen sein, dass das Schrankteil eine vorzugsweise ausziehbar ausgestaltete Schublade bereitstellt, die der Aufnahme von Peripheriegeräten wie z.B. einer Tastatur und/oder einer Maus dient. Dabei können derlei Peripheriegeräte über Funk mit dem Rechner kommunikationstechnisch gekoppelt sein. Alternativ kann auch eine Verkabelung vorgesehen sein, wobei in diesem Fall die Kabel über das den Monitor tragende Rohr und den Kabelraum bis hin zum Rechner geführt sind.

Gemäß einem weiteren Merkmal der Erfindung verfügt der Stationswagen über ein Energiemanagementsystem, das mit Akkus ausgerüstet ist. Insofern ist auch ein vom Stromnetz getrennter Betrieb der EDV-Anlage gestattet. Das Energiemanagementsystem kann im Kabelraum untergebracht sein. Alternativ ist ein das Energiemanagementsystem aufnehmendes Gehäuse vorgesehen, das unterseitig der Bodenplatte des Schrankteils angeordnet ist. Über entsprechende Durchbrüche in der Bodenplatte erfolgt ein kabelgeschützter Anschluss des Energiemanagementsystems an den im Staufach des Stationswagens angeordneten Rechner.

Mit der erfindungsgemäßen Ausgestaltung wird insgesamt ein Stationswagen bereitgestellt, der es dank seiner konstruktiven Ausgestaltung gestattet, eine EDV-Anlage samt ggf. vorhandener Peripheriegeräte in einer solchen Weise aufzunehmen, dass eine vollgekapselte Anordnung erreicht ist und insbesondere ansonsten störende Kabel vollständig geschützt untergebracht sind. Die Handhabung des Stationswagens ist damit insgesamt verbessert und es können Beeinträchtigungen durch ansonsten freiliegende Kabel vollends vermieden werden. Darüber hinaus ist ein unberechtigter Zugriff auf die Rechneranlage vermieden, was die Manipulationssicherheit verbessert. Zudem trägt die Entwicklung der gekapselten Ausführung des Monitors und der verdeckten Kabelführung den im Krankenhaus geforderten erhöhten Hygiene- und Reinigungsanforderungen Rechnung.

Weitere Merkmale und Vorteile der Erfindung ergeben sich aus der nachfolgenden Beschreibung anhand der Figuren. Dabei zeigen
- Fig. 1 bis 5: den erfindungsgemäßen Stationswagen gemäß einer ersten Ausführungsform in unterschiedlichen Ansichten und
- Fig. 6 bis 9: den erfindungsgemäßen Stationswagen gemäß einer zweiten Ausführungsform in unterschiedlichen Ansichten.

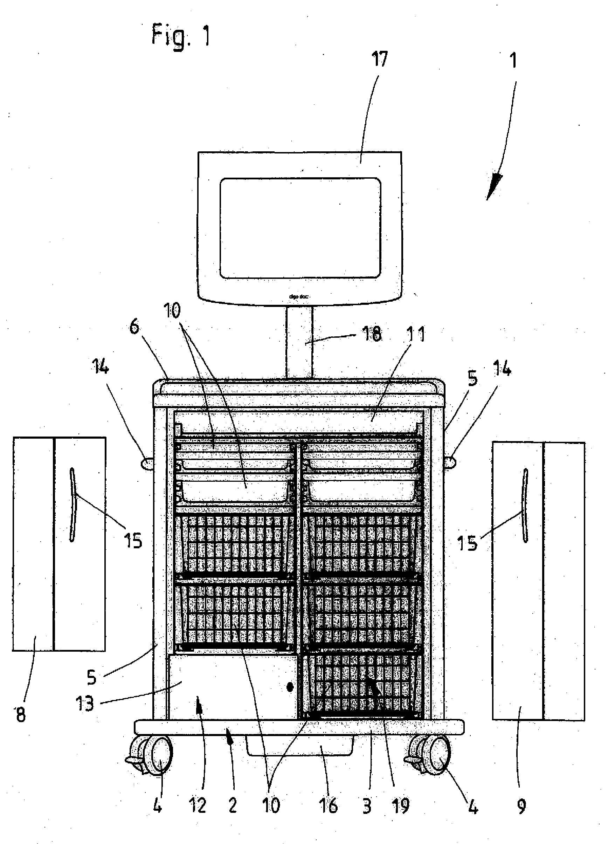

Fig. 1 lässt in einer Draufsicht von vorn den erfindungsgemäßen Stationswagen 1 gemäß einer ersten Ausführungsform erkennen.

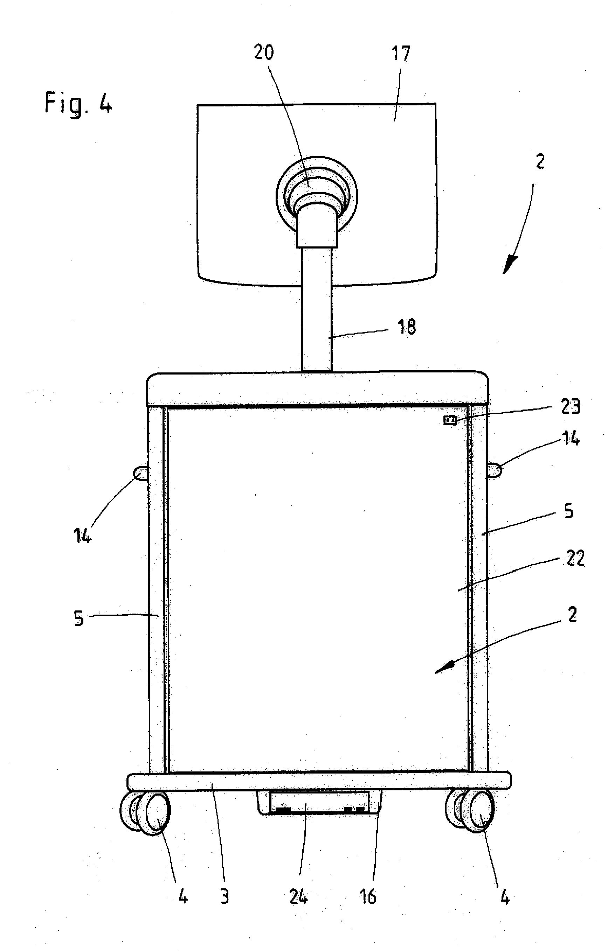

Der Stationswagen 1 verfügt über ein Schrankteil 2. Dieses Schrankteil 2 verfügt unterseitig über eine Bodenplatte 3 und oberseitig über eine Abdeckung 6. Es sind zudem Seitenwände 5 sowie eine Rückwand 22 vorgesehen, wobei die Rückwand 22 aus der rückwärtigen Ansicht des Stationswagens 1 nach Fig. 4 zu erkennen ist.

Die Bodenplatte 3 stützt sich über vier Tragrollen 4 ab, die bevorzugter Weise um ihre Hochachse verdrehbar ausgebildet sind. Mittels der Tragrollen 4 ist der Stationswagen 1 verfahrbar ausgebildet.

Das Schrankteil 2 stellt einen Stauraum 19 bereit. Dieser dient der Aufnahme von Utensilien, wobei für eine geordnete Unterbringung Körbe 10 vorgesehen sein können, wie dies die Ausführungsform nach Fig. 1 beispielhaft erkennen lässt.

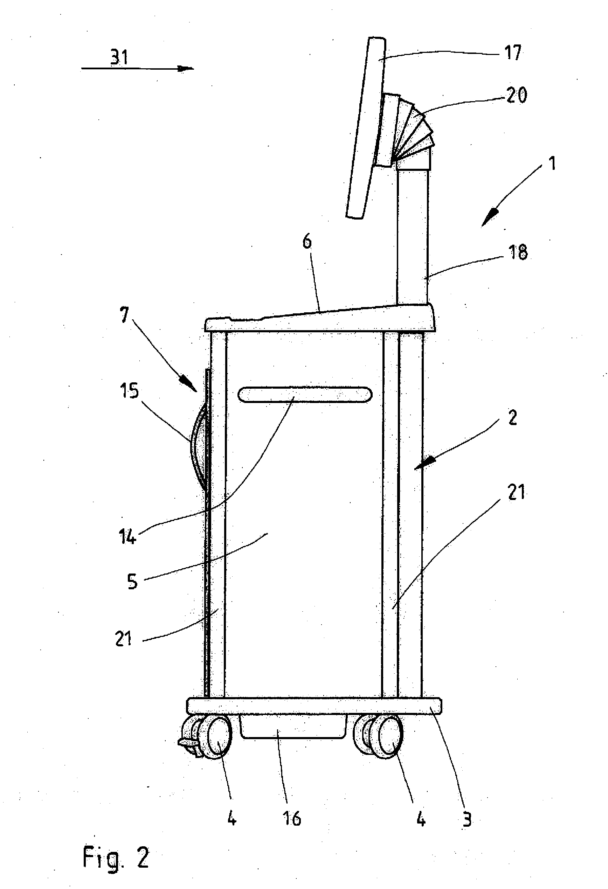

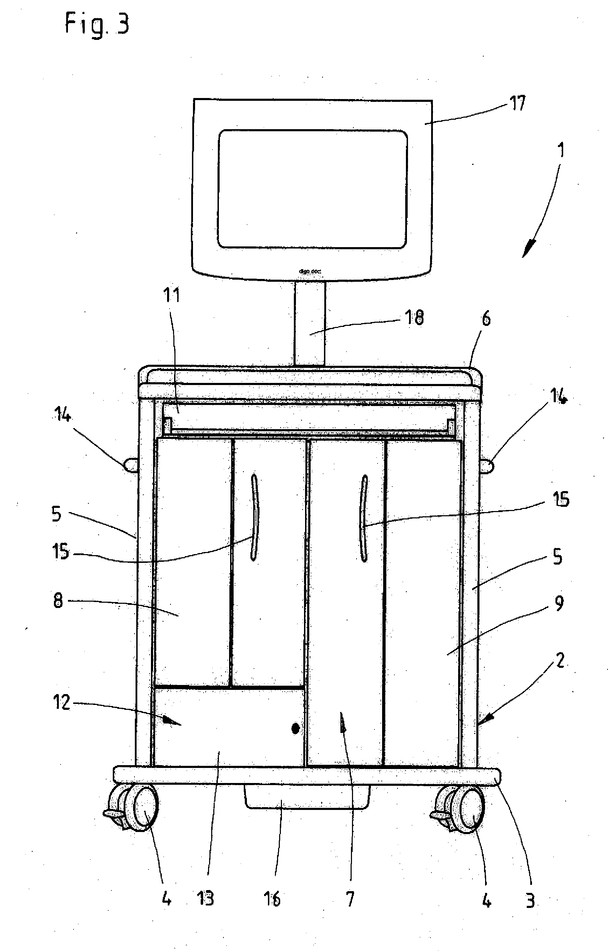

Das Schrankteil 2 verfügt frontseitig über eine Tür 7, die im gezeigten Ausführungsbeispiel über zwei Türflügel 8 und 9 verfügt, die jeweils eine Handhabe 15 in Form eines Griffes bereitstellen. Die Darstellung nach Fig. 1 zeigt die Tür 7 bzw. die Türflügel 8 und 9 in einem nicht montierten Zustand, was den frontseitigen Einblick in den Stauraum 19 gestattet. Die weiteren Figuren 2 und 3 zeigen die Tür 7 in ihrer ordnungsgemäß montierten und verschlossenen Stellung.

Wie insbesondere die Seitenansicht nach Fig. 2 erkennen lässt, können zur Abstützung der Abdeckung 6 gegenüber dem Bodenteil 3 Pfosten 21 vorgesehen sein. Diese können in die Seitenwand 5 integriert ausgebildet sein, oder die Seitenwand 5 erstreckt sich von einem Pfosten 21 zum nächsten.

Für eine verbesserte Handhabe im Zuge einer Verfahrbewegung verfügt der Stationswagen 1 über Griffe 14, die im gezeigten Ausführungsbeispiel von den Seitenwänden 5 getragen sind, wie dies insbesondere die Figuren 1 und 3 erkennen lassen.

Der Stauraum 19 stellt ferner ein separat ausgebildetes Staufach 12 bereit. Dieses ist frontseitig mittels einer Staufachtür 13 verschließbar ausgebildet. Um einen Zugriff auf das Staufach durch Unberechtigte zu vermeiden, können entsprechende Schließmechanismen zur Verrieglung der Staufachtür vorgesehen sein, beispielsweise in der Ausgestaltung eines Profilzylinderschlosses.

Der Stationswagen 1 stellt ferner einen Monitor 17 bereit. Dieser ist vorzugsweise verschwenk- und/oder verdrehbar von einem Rohr 18 gehalten. Zur verschwenkbaren Anordnung des Monitors 17 am Rohr 18 kann ein entsprechendes Gelenk vorgesehen sein, das im gezeigten Ausführungsbeispiel nach Fig. 2 durch eine Tülle 20 oder Manschette umhüllt und damit abgedeckt ist. Dabei besteht die Tülle 20 oder Manschette vorzugsweise aus Kunststoff, wohingegen das Rohr 18 bevorzugterweise aus Aluminium oder einer Aluminiumlegierung gebildet ist.

Das Schrankteil 2 verfügt erfindungsgemäß über einen Kabelraum 32. Dieser ist in Beschickungsrichtung 31 des Schrankteils 2 hinter dem Stauraum 19 liegend ausgebildet, wie insbesondere die rückwärtige Ansicht des Stationswagens 1 nach Fig. 4 erkennen lässt. Dabei sind der Stauraum 19 und der Kabelraum 32 durch eine Zwischenwand 25 voneinander getrennt. Aufgrund dessen ist ein frontseitiger Zugriff auf den Kabelraum 32 durch den Stauraum 19 hindurch nicht möglich.

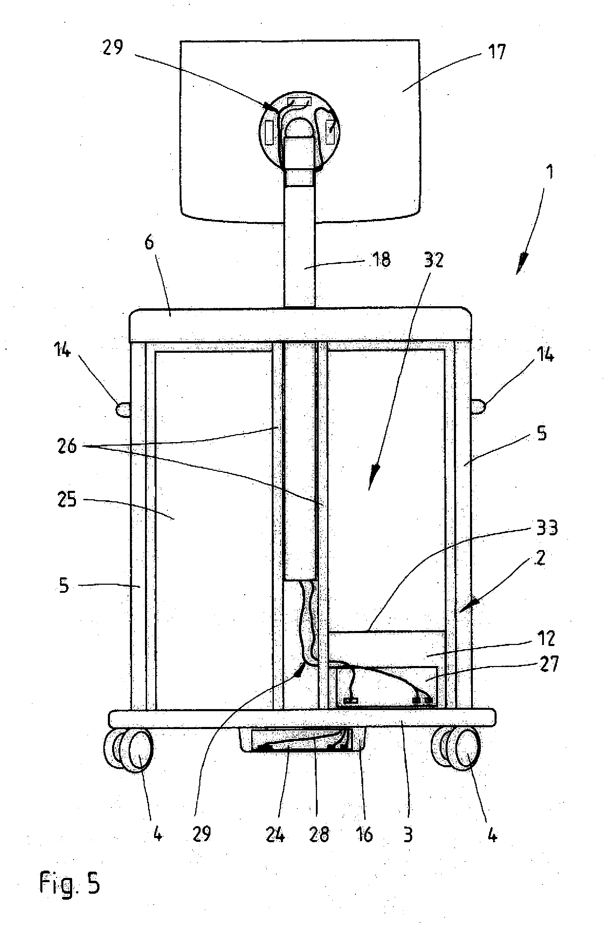

Das vom Stauraum 19 bereitgestellte Staufach 12 stellt einen Zugang zum Kabelraum 32 bereit, was sich aus der Darstellung nach Fig. 5 ergibt, wonach der Zugang durch eine Öffnung 33 in der Zwischenwand 25 gebildet ist.

Wie die Darstellung nach Fig. 5 ferner erkennen lässt, dient das vom Stauraum 19 bereitgestellte Staufach der Aufnahme eines Rechners 27. Dieser ist über eine Verkabelung 29 mit dem Monitor 17 gekoppelt. Dabei wird die Verkabelung 29 durch die Öffnung 33 in den erfindungsgemäß vorgesehenen Kabelraum 32 und von dort aus durch das Rohr 18 bis hin zu den Anschlüssen des Monitors 17 geführt. Dabei zeigt Fig. 5 der besseren Darstellung wegen nicht die aus den Figuren 3 und 4 erkennbare Tülle 20 oder Manschette, die zum einen der Abdeckung des den Monitor 17 mit dem Rohr 18 verbindenden Gelenks, sondern auch der Abdeckung der monitorseitigen Kabelanschlüsse dient.

Das Rohr 18 ragt mit seinem monitorentfernten Ende in den Kabelraum 32 hinein, ist also im endmontierten Zustand des Stationswagens 1 verwenderseitig weder zugänglich noch sichtbar. Es ist von Führungsstegen 26 oder -wänden gehalten, die darüber hinaus eine Höhenverstellung des Rohres 18 und damit des Monitors 17 relativ gegenüber dem Schrankteil 2 ermöglichen.

Im endfertig montierten Zustand ist das Schrankteil 2 und damit der Kabelraum 32 rückwärtig mittels einer Rückwand 22 verschlossen, wie dies die Darstellung nach Fig. 4 erkennen lässt. Dabei stellt die Rückwand 22 einen Stecker 23 integrativ bereit, der als Zentralstecker für die vom Stationswagen 1 aufgenommene EDV-Anlage dient und für den Anschluss an eine Stromversorgung und/oder ein Ladegerät vorgesehen ist.

Der Stationswagen 1 verfügt des Weiteren über ein Energiemanagementsystem 24, das im gezeigten Ausführungsbeispiel von einem Gehäuse 16 aufgenommen ist, das unterseitig der Bodenplatte 3 am Schrankteil 2 angeordnet ist. Das Energiemanagementsystem 24 kann zum Zwecke der Stromversorgung der EDV-Anlage über austauschbare Akkus verfügen, so dass auch ein Betrieb der EDV-Anlage ohne den direkten Anschluss an eine Stromversorgung gestattet ist.

Das Energiemanagementsystem 24 ist über eine Verkabelung 28 mit der EDV-Anlage verbunden, wobei die Verkabelung 28 durch in den Figuren nicht näher dargestellte Öffnungen in der Bodenplatte 3 in den Kabelraum 32 hineingeführt ist.

Zur Aufnahme einer Tastatur, einer Maus und/oder dgl. Peripheriegeräte kann der Stationswagen 1 über eine frontseitig zugängliche Schublade 11 verfügen, wie sich dies beispielsweise aus der Darstellung nach Fig. 1 oder 3 ergibt. Derartige Peripheriegeräte können über Funk mit dem Rechner 27 in kommunikationstechnischer Verbindung stehen. Denkbar ist aber auch eine kabelgestützte Verbindung, wobei in diesem Fall die Kabel der Peripheriegeräte über eine entsprechende Öffnung im Rohr 18 in selbiges geführt und in den Kabelraum 32 verlegt sind.

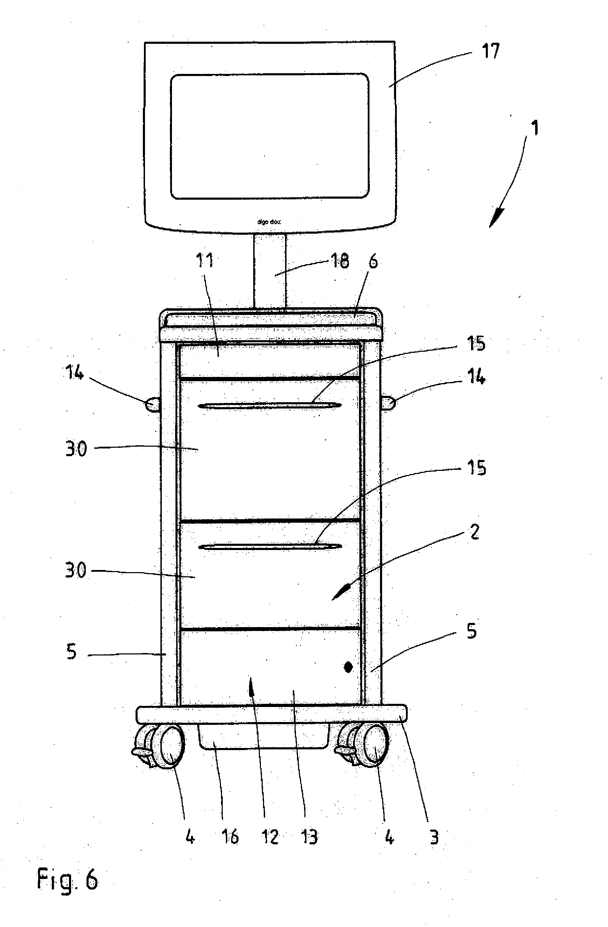

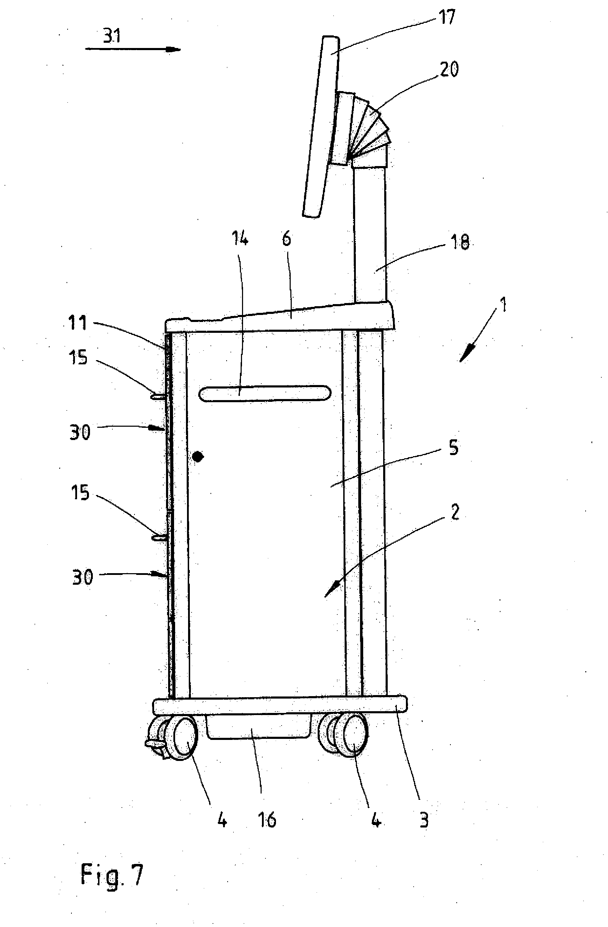

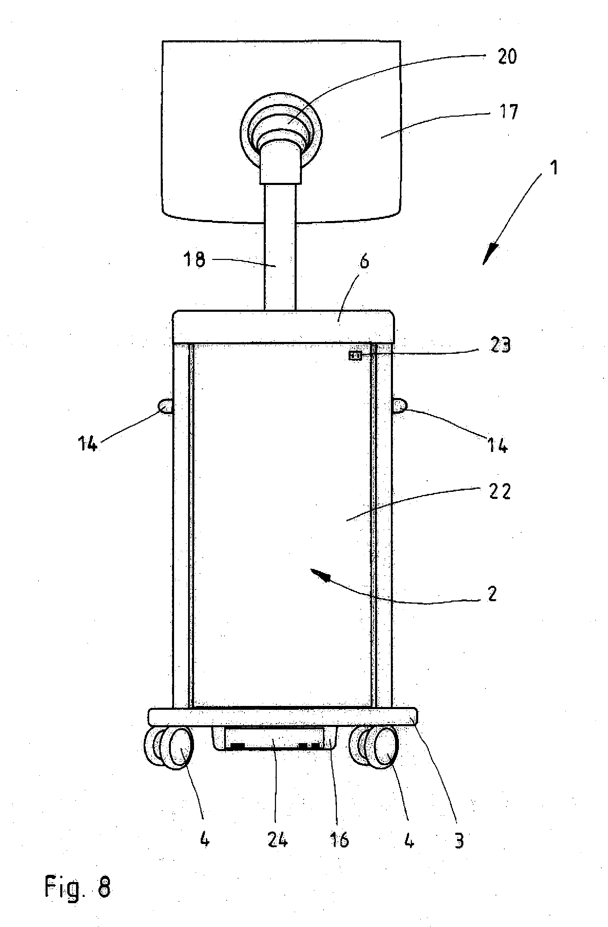

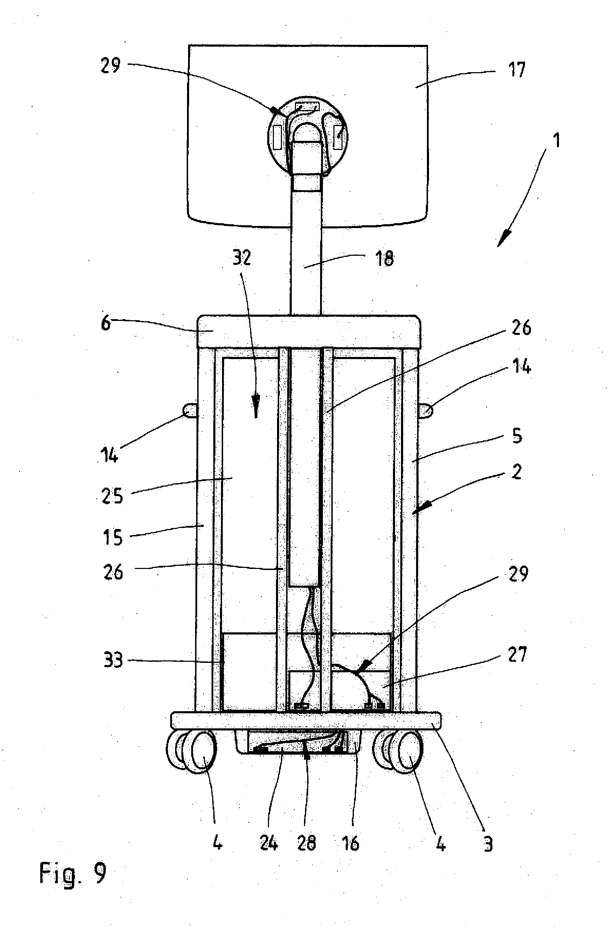

Die weiteren Figuren 6 bis 9 zeigen ein zweites Ausführungsbeispiel der Erfindung, wobei der hier dargestellte Stationswagen 1 anstelle eines über eine Tür 7 zugänglichen Stauraums 19 einen mit Schubladen 30 bestückten Stauraum 19 zeigt. Ansonsten stellt der Stationswagen 1 in der schon vorbeschriebenen Weise eine gekapselte Konstruktion bereit, dergemäß eine vollständig abgedeckte Kabelführung bezüglich der EDV-Anlage erreicht ist.

### Bezugszeichenliste

- 1: Stationswagen
- 2: Schrankteil
- 3: Bodenplatte
- 4: Tragrolle
- 5: Seitenwand
- 6: Abdeckung
- 7: Tür
- 8: Türflügel
- 9: Türflügel
- 10: Korb
- 11: Schublade
- 12: Staufach
- 13: Staufachtür
- 14: Griff
- 15: Handhabe
- 16: Gehäuse
- 17: Monitor
- 18: Rohr
- 19: Stauraum
- 20: Tülle
- 21: Pfosten
- 22: Rückwand
- 23: Stecker
- 24: Energiemanagementsystem
- 25: Zwischenwand
- 26: Führungssteg
- 27: Rechner
- 28: Verkabelung
- 29: Verkabelung
- 30: Schublade
- 31: Beschickungsöffnung
- 32: Kabelraum
- 33: Öffnung

## Patentansprüche

1. Stationswagen, insbesondere Visiten- und Pflegewagen für Krankenhäuser, Pflegeheime und sonstige Einrichtungen des Gesundheitswesens, mit einem fahrbar ausgebildeten Schrankteil (2), wobei das Schrankteil (2) einen frontseitig zugänglichen Stauraum (19) zur Aufnahme von Utensilien und einen in Beschickungsrichtung (31) des Stauraums (19) dahinterliegenden und vom Stauraum (19) durch eine Zwischenwand (25) getrennten Kabelraum (32) aufweist, wobei der Stauraum (19) ein Staufach (12) bereitstellt, das einen Zugang zum Kabelraum (32) aufweist, **gekennzeichnet durch** ein am Schrankteil (2) angeordnetes und von außen zugängliches Energiemanagementsystem (24), wobei das Energiemanagementsystem (24) gekapselt ausgebildet und unterseitig einer Bodenplatte (3) des Schrankteils (2) angeordnet ist und **durch** einen verschwenkbar am Schrankteil (2) angeordneten Monitor (17), wobei der Monitor (17) von einem Rohr (18) getragen ist, das mit seinem Monitor entfernten Ende in den Kabelraum (32) hineinragt, wobei der Monitor (17) unter Zwischenordnung eines Gelenks am Rohr (18) angeordnet ist, wobei das Gelenk **durch** eine Tülle (20) abgedeckt ist.

2. Stationswagen nach Anspruch 1, **dadurch gekennzeichnet, dass** das Schrankteil (2) rückseitig eine den Kabelraum (32) verschließende Rückwand (22) aufweist.

3. Stationswagen nach Anspruch 2, **dadurch gekennzeichnet, dass** die Rückwand (22) einen von außen zugänglichen Stromanschlussstecker (23) bereitstellt.

4. Stationswagen nach Anspruch 1 oder 2, **dadurch gekennzeichnet, dass** das Staufach (12) stauraumseitig der Bodenplatte (3) ausgebildet ist.

5. Stationswagen nach Anspruch 4, **dadurch gekennzeichnet, dass** die Bodenplatte (3) im Überdeckungsbereich mit dem Staufach (12) einen Durchbruch aufweist.

## Claims

1. A trolley, in particular ward trolley and care trolley for hospitals, nursing homes and other health care establishments, comprising a movable cabinet element (2), wherein the cabinet element (2) comprises a storage space (19) which is accessible from the front side and which serves to receive utensils and a cable space (32) which is located behind the storage space (19) in the loading direction (31) of the same one and which is separated from the storage space (19) by a partition wall (25), wherein the storage space (19) provides a storage compartment (12) which comprises an access to the cable space (32), **characterized by** an energy management system (24) accessible from outside and arranged on the cabinet element (2), wherein the energy management system (24) is designed in an encapsulated manner and arranged on the underside of a bottom plate (3) of the cabinet element (2), and by a monitor (17) mounted in a swiveling manner on the cabinet element (2), wherein the monitor (17) is carried by a tube (18) which projects with its distant end with respect to the monitor into the cable space (32), wherein the monitor (17) is arranged on the tube (18) with interposition of an articulation, wherein the articulation is covered by a grommet (20).

2. A trolley according to claim 1, **characterized in that** the cabinet element (2) comprises a rear wall (22) which closes the rear side of the cable space (32).

3. A trolley according to claim 2, **characterized in that** the rear wall (22) provides a power connection plug (23) accessible from outside.

4. A trolley according to claim 1 or 2, **characterized in that** the storage compartment (12) is formed on the side of the storage space of the bottom plate (3).

5. A trolley according to claim 4, **characterized in that** the bottom plate (3) comprises an opening in the overlapping area with the storage compartment (12).

## Revendications

1. Chariot, notamment chariot de visites et de soins, destiné à des hôpitaux, des établissements de soins ou à d'autres établissements de santé, comprenant un élément d'armoire mobile (2), l'élément d'armoire (2) comprenant un espace de rangement (19), qui est accessible à partir du côté avant et qui sert à recevoir des ustensiles, et un espace de câbles (32), qui est disposé derrière l'espace de rangement (19) dans la direction de chargement (31) de celui-ci et est séparé de l'espace de rangement (19) par une paroi intermédiaire (25), l'espace de rangement (19) fournissant un compartiment de rangement (12), qui comprend un accès à l'espace de câbles (32), **caractérisé par** un système de gestion de l'énergie (24) accessible de l'extérieur et disposé sur l'élément d'armoire (2), le système de gestion de l'énergie (24) étant configuré sous forme encapsulée et étant disposé sur la face inférieure d'une plaque de fond (3) de l'élément d'armoire (2), et par un moniteur (17) disposé de manière pivotante sur l'élément d'armoire (2), le moniteur (17) étant porté par un tube (18), qui fait saillie avec son extrémité distale par rapport au moniteur dans l'espace de câbles (32), le moniteur (17) étant disposé sur le tube (18) avec interposition d'une articulation, l'articulation étant couvert par une douille (20).

2. Chariot selon la revendication 1, **caractérisé en ce que** l'élément d'armoire (2) comprend une paroi arrière (22), qui ferme le côté arrière de l'espace de câbles (32).

3. Chariot selon la revendication 2, **caractérisé en ce que** la paroi arrière (22) fournit une prise d'alimentation en courant (23) accessible de l'extérieur.

4. Chariot selon la revendication 1 ou la revendication 2, **caractérisé en ce que** le compartiment de rangement (12) est formé sur le côté de l'espace de rangement de la plaque de fond (3).

5. Chariot selon la revendication 4, **caractérisé en ce que** la plaque de fond (3) comprend une percée dans la zone de chevauchement avec le compartiment de rangement (12).
